# EUROPEAN PATENT APPLICATION

(11) **EP 4 595 893 A1**
(43) Date of publication of application: **06.08.2025**
(21) Application number: 22961125.6
(22) Date of filing: 19.12.2022
(51) Int. Cl.: A61B 8/00, A61B 5/00

(54) **HELICAL SCAN PHOTOACOUSTIC-ULTRASONIC ENDOSCOPE**

(30) Priority: 30.09.2022 KR 20220125819; 16.12.2022 KR 20220177398
(71) Applicant: Ulsan National Institute of Science and Technology (UNIST), Eonyang-eup Ulju-gun Ulsan 44919 (KR)
(72) Inventor: YANG, Joon-Mo, Ulju-gun, Ulsan 44919 (KR)
(74) Representative: Studio Torta S.p.A.
(86) International application number: PCT/KR2022/020692
(87) International publication number: WO 2024/071526

(57) **Abstract**

The present disclosure relates to a photoacoustic-ultrasonic endoscope capable of three-dimensional helical scanning, including a probe and a driving unit providing rotational power and pullback translational motion to the probe, wherein the probe includes a catheter tubing extending in one direction and having an interior that is hollow, one end of the catheter tubing being closed and the other end of the catheter tubing being open, a fastening nut that is fastened to the other end of the catheter tubing and has a through hole corresponding to the interior of the catheter tubing, a pullback shaft extending into the interior of the catheter tubing through the through hole of the fastening nut and having an interior that is hollow, and a radial shaft seal interposed between the fastening nut and the pullback shaft and surrounding the pullback shaft around a central axis of the through hole of the fastening nut.

## Description

### Technical Field

The present disclosure relates to a medical tomography endoscopy device that is implemented in the form of a thin and long probe like an ultrasonic endoscope currently used in clinical practice and may be inserted into a subject to provide a tomographic image of the surrounding area.

### Background Art

The present disclosure relates to so-called integrated photoacoustic and ultrasonic endoscopy (PAE-EUS) technology that may simultaneously provide photoacoustic imaging information while maintaining the function of conventional endoscopic ultrasound.

### Disclosure of Invention

### Technical Problem

Photoacoustic-ultrasonic endoscopy devices for digestive endoscopy implemented according to conventional technology have the following problems. 1) A rotating body, which consists of a torque coil, sensor and optical system, which performs mechanical scanning inside the probe, may only perform a simple two-dimensional rotational motion with respect to a catheter tubing (i.e., an outer shell) and a radial shaft seal, which are two core elements that have to be provided inside the probe to contain a matching fluid, and thus, only two-dimensional tomographic images are provided. 2) Because the process of separation and reconnection between the probe and a driving unit is not easy, difficult optical alignment is required when the user replaces the probe, and it takes a considerable amount of time to complete all the connections of electrical paths that allow electrical signals to pass. In particular, when the optical alignment process is performed on an endoscopic device based on single-mode optical fiber, an even longer optical alignment time is required.

### Solution to Problem

The present disclosure provides an embodiment, in which, based on a standard ceramic ferrule with a diameter of 2.5 mm, which is commonly used for the fixation and termination of a bare optical fiber at the end of an optical fiber cable, and an FC/PC connector and a related dedicated adapter manufactured to fit the standard ceramic ferrule, both precise "optical alignment" and "electrical path connection", which are the most essential requirements for satisfactory achievement of the technology, may be completed immediately after the probe is fastened to the driving unit.

In addition, by placing a radial shaft seal and a pullback shaft having a predetermined length at the probe side rather than the driving unit side, a photoacoustic-ultrasonic endoscopic probe, which is capable of performing three-dimensional helical scanning without any leakage of the fluid filled in the probe although a torque coil and scanning tip rotating inside the probe perform a rotational motion as well as a pullback translational motion, and in which the separation and connection between the probe and the driving unit are easy, is derived. In addition, structure of a dedicated driving unit capable of driving the photoacoustic-ultrasonic endoscopic probe effectively is derived. That is, a device including a rotating opto-electromagnetic coupling unit and a mechanical module is derived, wherein the rotating opto-electromagnetic coupling unit may smoothly exchange photoacoustic-ultrasonic electrical signals, which are generated as a result of the transmission and firing of laser beams required for photoacoustic imaging as well as the electrical pulses required for ultrasound imaging, are detected by an ultrasonic transducer, and then are transmitted toward the proximal end, and the mechanical module provides the rotational and pullback power necessary to enable the aforementioned three-dimensional helical scan within a catheter.

The descriptions given above already include a new and progressive concept that has not been shown in the prior art, and thus, they are described in one specification in order to more easily explain the effects that their combined use may provide.

According to an aspect of the present disclosure, a helical scan photoacoustic-ultrasonic endoscope including a probe and a driving unit providing rotational power and pullback translational motion to the probe, wherein the probe includes a catheter tubing extending in one direction and having an interior that is hollow, one end of the catheter tubing being closed and the other end of the catheter tubing being open, a fastening nut that is placed to the aforementioned other end of the catheter tubing and has a through hole corresponding to the interior of the catheter tubing so that it could be used to connect the probe to driving unit, a pullback shaft that extends into the interior of the catheter tubing through the through hole of the fastening nut and has an interior that is hollow, and a shaft seal that is interposed between the fastening nut and the pullback shaft and surrounds the pullback shaft around a central axis of the through hole of the fastening nut, is provided.

The driving unit may include a rotary transformer and a motorized linear stage, which could be mechanically connected to the pullback shaft.

The radial shaft seal may shield the interior of the catheter tubing from the outside.

The helical scan photoacoustic-ultrasonic endoscope may further include a probe-side optical fiber that passes through the interior of the pullback shaft and extends in a direction toward the one end of the catheter tubing, a torque coil that surrounds the probe-side optical fiber and is engaged with the pullback shaft in a way that it passes through the pullback shaft and extends in the direction toward the one end of the catheter tubing, and a scanning tip connected to a part of the torque coil in the direction toward the one end of the catheter tubing. The pullback shaft, the probe-side optical fiber, the torque coil, and the scanning tip are relatively movable with respect to the catheter tubing, the fastening nut, and the radial shaft seal in a direction in which the catheter tubing extends and in an opposite direction that is opposite to the direction. The helical scan photoacoustic-ultrasonic endoscope may further include a matching fluid filling the interior of the catheter tubing.

The helical scan photoacoustic-ultrasonic endoscope may further include a connector connected to an end of the pullback shaft in a direction facing away from the one end of the catheter tubing.

The connector may be located in the direction facing away from the one end of the catheter tubing with respect to the shaft seal.

The helical scan photoacoustic-ultrasonic endoscope may further include a micro coaxial cable electrically connected to the scanning tip and extending to the connector through the interior of the torque coil, wherein the connector may include a ferrule having a central hole through which the probe-side optical fiber passes, a connector housing surrounding at least a part of the ferrule and having electrical conductivity, a connector nut physically contacting the connector housing and having electrical conductivity, and a connector insulating member interposed between the ferrule and the connector housing to electrically insulate the ferrule from the connector housing, wherein a core part of the micro coaxial cable may be electrically connected to the ferrule, and a shield part of the micro coaxial cable may be electrically connected to the connector housing. The driving unit may include an adapter, wherein the adapter may include a split mating sleeve into which a part of the ferrule is inserted, an adapter frame surrounding at least a part of the ferrule and having electrical conductivity, and an adapter insulating member interposed between the split mating sleeve and the adapter frame to electrically insulate the split mating sleeve from the adapter frame.

Other aspects, features and advantages other than those described above will become apparent from the following detailed description, claims and drawings for practicing the disclosure.

### Advantageous Effects of Invention

According to the present disclosure, when a photoacoustic-ultrasonic endoscope is implemented in a form in which a probe and a driving unit may be able to be separated or connected each other based on a standard FC/PC or FC/APC connector manufactured for existing optical communication technology, very stable and precise optical coupling may be quickly achieved at an optical coupling point even when applying a single-mode optical fiber within a very light and compact size. Therefore, not only may the procedure of related medical procedures in the future be conducted more conveniently and smoothly, but three-dimensional images obtained through the photoacoustic-ultrasonic endoscope may also be much more accurate and reliable. In addition, when a connector part is manufactured by the proposed method, the cost required for production is very low and it is possible to replace only the probe part when the probe is damaged. Thus, patients who need related examinations may benefit from the related technology at a lower cost, and the connection part of the probe may also be implemented very small and light, so that the probability of damage is significantly reduced even when the probe is dropped during the medical procedures.

The concept proposed by the present disclosure is mainly proposed as a target for endoscopes for diagnosing digestive diseases, but the concept may be applied to various fields of endoscopy, such as diagnosing cardiovascular diseases.

However, the scope of the present disclosure is not limited by these effects.

### Brief Description of Drawings

FIG. 1 is a schematic diagram showing the overall configuration and operating principle of a helical scan photoacoustic-ultrasonic endoscope according to an embodiment of the present disclosure.
FIGS. 2A and 2B are schematic diagrams showing the internal state of a driving unit included in the helical scan photoacoustic-ultrasonic endoscope of FIG. 1, before and after a pullback motion.
FIGS. 3A and 3B are perspective views showing the configuration of a probe included in the helical scan photoacoustic-ultrasonic endoscope of FIG. 1.
FIG. 4A is a perspective view schematically illustrating typical optical fiber-related components that may be used in the field of optical communications.
FIGS. 4B to 4G are schematic diagrams showing the principles of optical coupling and electrical path formation between a probe and a driving unit included in the helical scan photoacoustic-ultrasonic endoscope of FIG. 1.
FIG. 5 is a schematic diagram showing a method of achieving high-efficiency optical coupling in optical coupling in a helical scan photoacoustic-ultrasonic endoscope according to an embodiment of the present disclosure.
FIGS. 6A and 6B are schematic diagrams showing a method of implementing an optical input part in a helical scan photoacoustic-ultrasonic endoscope according to an embodiment of the present disclosure.
FIG. 7 is a schematic diagram showing the possibility of changing the position of a rotary transformer in a helical scan photoacoustic-ultrasonic endoscope according to an embodiment of the present disclosure.

### Best Mode for Carrying out the Invention

As the present disclosure allows for various changes and numerous embodiments, particular embodiments will be illustrated in the drawings and described in detail in the written description. Hereinafter, effects and features of the present disclosure and a method for accomplishing them will be described more fully with reference to the accompanying drawings, in which embodiments of the present disclosure are shown. The present disclosure may, however, be embodied in many different forms and should not be construed as limited to the embodiments set forth herein.

Embodiments of the present disclosure will be described below in more detail with reference to the accompanying drawings. Those components that are the same as or are in correspondence with each other are rendered the same reference numeral regardless of the figure number, and redundant explanations are omitted.

In the following embodiments, it will be understood that although the terms "first," "second," etc. may be used herein to describe various components, these components should not be limited by these terms. These components are only used to distinguish one component from another.

In the following embodiments, the singular expression includes the plural unless the context clearly indicates otherwise.

In the following embodiments, it will be further understood that the terms "includes", "has", "including" and/or "having" used herein specify the presence of stated features or components, but do not preclude the presence or addition of one or more other features or components.

In the following embodiments, when a component is "connected" to another component, the component may be directly connected to the other component, or may be indirectly connected to the other component through another component.

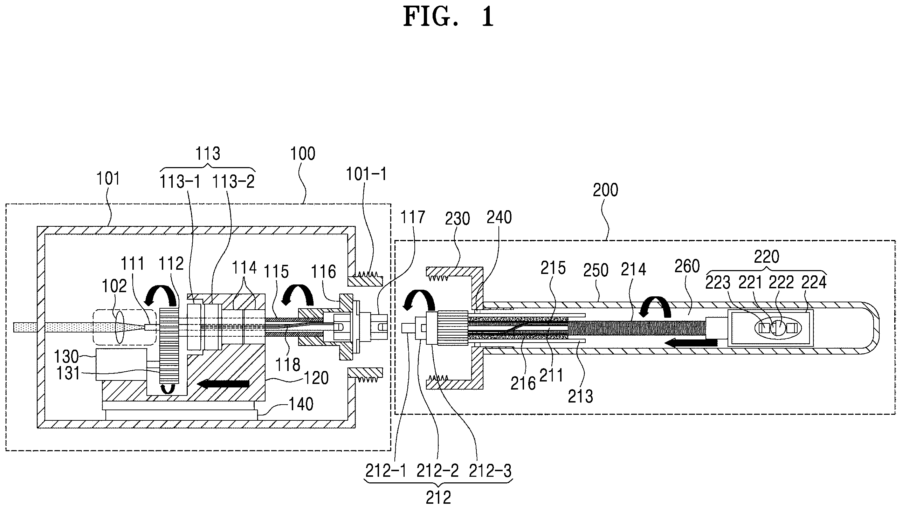

FIG. 1 is a schematic diagram showing the overall configuration and operating principle of a helical scan photoacoustic-ultrasonic endoscope according to an embodiment of the present disclosure.

Referring to FIG. 1, the helical scan photoacoustic-ultrasonic endoscope according to an embodiment is largely composed of a probe 200 that is inserted into a subject to perform photoacoustic-ultrasonic imaging, and a driving unit 100 that provides laser pulses and electric pulses necessary for the probe 200 to perform the photoacoustic-ultrasonic imaging and also provides rotational power and pullback translational motion to the probe 200. When a photoacoustic-ultrasonic imaging procedure is to be performed with an endoscopic device implemented according to the present disclosure, an FC/PC connector 212 and a fastening nut 230 of the probe 200 have to be sequentially fastened and fixed to an FC/PC adapter 117 inside the driving unit 100 and a fastening male screw 101-1 provided in a driving unit case 101, respectively. The driving unit case 101 accommodates a through-type shaft 115, a driving gear 112, a base frame 120, and a motorized linear stage 140 as described below.

The overall operating principle and the roles of each element will be explained assuming that the two parts are fastened to each other in the above-described manner.

In general, integrated photoacoustic-ultrasonic endoscopic devices developed in the relevant technical field fire a predetermined number of ultrasonic pulses and laser pulses toward a target or subject to be examined in an alternating way within a very fast time while a scanning tip rotates, detect a one-dimensional response signal (either an ultrasonic or photoacoustic response signal) called the "A-line" that propagates from the target or subject immediately after each pulse is fired, and collect all of these individually A-line signals to form a two-dimensional ultrasonic and photoacoustic fusion image.

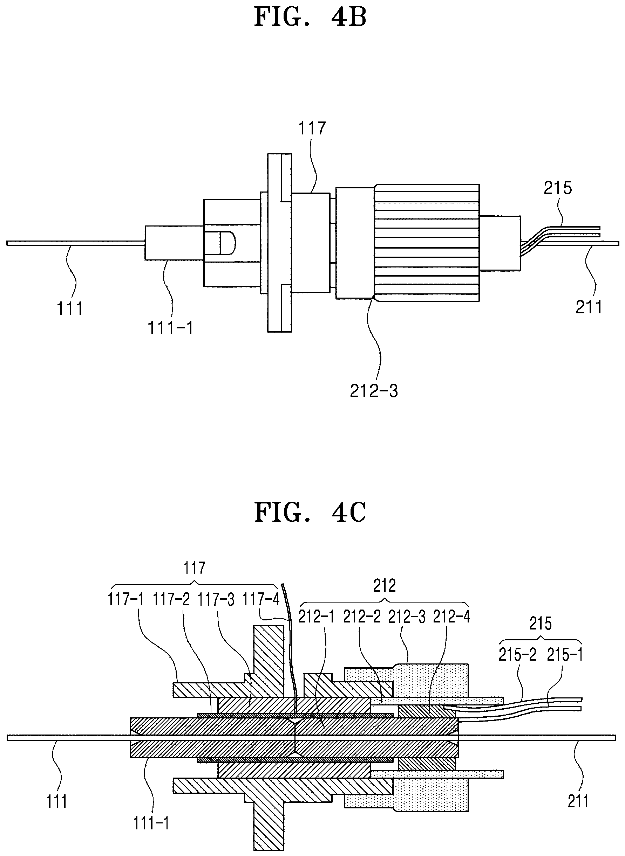

First, a process of obtaining a single A-line "ultrasonic signal" will be described. A high-voltage electric pulse having a very short duration, generated from an ultrasonic pulser/receiver (not shown) provided separately from the present disclosure, is sent to a second coil part 113-2, which is one coil part of a rotary transformer 113, and then is transferred to a first coil unit 113-1, which is the other coil part of the rotary transformer 113, by an electromagnetic induction phenomenon that occurs immediately thereafter. Next, the electric pulse flows into a transducer 223 through a micro coaxial cable 215 provided in a pullback shaft 213 and a torque coil 214 at the probe 200 side, via an electric coupling path between the FC/PC adapter 117 and the FC/PC connector 212, which will be described below, and a shaft electric wire 118 inside a shaft, which is directly connected to the first coil part 113-1. The coaxial cable 215 includes a core part 215-1 (see FIGS. 4B and 4C) and a shield part 215-2 (see FIGS. 4B and 4C), which are coaxially configured each other, but in FIG. 1, they are illustrated as two wires going parallel for convenience. The shaft electric wire 118 inside the shaft is also illustrated as two wires, and may also have a coaxial cable structure having a coaxial core part and a shield part. The transducer 223 that receives the electric pulse converts the electric pulse into an ultrasonic wave according to a piezoelectric effect and transmits the ultrasonic wave toward the subject. Then, some of the ultrasonic waves reflected inside the tissue of the subject are detected by the transducer 223 and flow into the ultrasonic pulser/receiver (not shown) in the reverse order of the aforementioned process and are finally recorded by a data collection device provided separately from the present disclosure.

In contrast to the above, a process of obtaining a single A-line "photoacoustic signal" will be described with reference to the same drawing. An optical pulse having a very short duration, generated from a pulsed light source (not shown) of a nanosecond level, provided separately from the present disclosure, is transmitted to the driving unit 100 via an optical fiber (not shown) provided separately, and then is fired toward a driving unit-side optical fiber 111 located in the through-type shaft 115 extending in one direction through an optical input unit 102. Through this, the optical pulse is moved to the FC/PC adapter 117 that is precisely fixed along the central axis of the through-type shaft 115 extending in one direction by an FC/PC adapter frame 117-1. For reference, the adapter 117 is mechanically (directly or indirectly) connected to the end of the through-type shaft 115 in a direction toward the probe 200 and has a through hole corresponding to the interior of the through-type shaft 115. Afterwards, the corresponding light pulse travels along a probe-side optical fiber 211 distributed from the entrance of the FC/PC connector 212 to a scanning tip 220 through a very well-aligned optical path provided by the FC/PC adapter 117, and is finally emitted through a lens unit 221. In this case, the size of an emitted beam (the emitted light pulse) may be appropriately set by the lens unit 221, and the beam is reflected by an optical reflector 222 and sent to a tissue to be examined to generate a photoacoustic wave. A part of the photoacoustic wave generated in the tissue to be examined is propagated toward the transducer 223, and when the part of the photoacoustic wave is detected by the transducer 223, the detected part of the photoacoustic wave is converted into an electric signal. This electric signal also flows to the ultrasonic pulser/receiver (not shown) along the same path as the aforementioned "detected ultrasonic signal" and is finally recorded by the data collection device provided separately from the present disclosure.

The ultrasonic pulser/receiver (not shown) mentioned above is not included in the drawing of the present disclosure because it is an element that is obviously used in the related field, and may be included in the driving unit 100 or provided as a separate element outside the driving unit 100.

The processes of obtaining the A-line "ultrasonic signal" and the A-line "photoacoustic signal" have been described above, and now the mechanical operating principle will be described.

The probe 200 may include a catheter tubing 250, a fastening nut 230, a pullback shaft 213, and a radial shaft seal 240.

The catheter tubing 250 has a structure in which the inside is hollow and extends in one direction (left and right directions for convenience in FIG. 1), and one end (for example, the right end as exemplarily illustrated in FIG. 1) is closed and the other end (for example, the left end as exemplarily illustrated in FIG. 1) is open. The catheter tubing 250 may be flexible. The fastening nut 230 is fastened to the other end of the catheter tubing 250 and has a corresponding through hole to the interior of the catheter tubing 250.

The pullback shaft 213 extends into the interior of the catheter tubing 250 through the through hole of the fastening nut 230 and has a through hole extending along a central axis of the pullback shaft 213, and thus may have an overall hollow cylindrical shape. Accordingly, the probe-side optical fiber 211 may pass through the interior of the pullback shaft 213 and extend in a direction toward one end of the catheter tubing 250. The torque coil 214 surrounding the probe-side optical fiber 211 may also pass through the interior of the pullback shaft 213 and extend in the direction toward one end of the catheter tubing 250. The scanning tip 220 may be connected to the probe-side optical fiber 211 and a part of the torque coil 214 in the direction toward one end of the catheter tubing 250. For reference, in FIG. 1, in order to schematically illustrate the probe-side optical fiber 211, the coaxial cable 215, etc., which are surrounded by the torque coil 214, a part of the torque coil 214 (in the direction toward the other end of the catheter tubing 250) is illustrated in a cross-sectional view.

The radial shaft seal 240 is interposed between the fastening nut 230 and the pullback shaft 213 and may surround (or completely surround or encircle) the pullback shaft 213 around the central axis of the through hole of the fastening nut 230.

A detailed description of each of the components of the probe 200 will be given below. However, the probe 200 may further include other additional components.

In a state where the probe 200 is fastened to the driving unit 100 through the aforementioned method, the rotational power generated by a motor 130 is transmitted to the driving gear 112 side through a pinion gear 131. The transmitted rotational power is transferred to the through-type shaft 115 directly or indirectly connected to the driving gear 112, to an FC/PC adapter mount 116, to the FC/PC adapter 117, and to the FC/PC connector 212 connected to the pullback shaft 213 located at the other end of the catheter tubing 250, and is continuously transmitted to the scanning tip 220 through the pullback shaft 213 which is directly or indirectly connected to the FC/PC connector 212, and the torque coil 214. As a result, the mentioned elements are simultaneously rotated in the direction of the arrow indicating a rotational motion shown in FIG. 1, and as soon as a predetermined number of photoacoustic and ultrasonic A-line signals are acquired for each rotation, they are each presented as a single cross-sectional image on a monitor screen (not shown).

A ball bearing module 114, which may be composed of a plurality of ball bearing rows and is mounted on the base frame 120, provides a mechanical condition in which the axis of the through-type shaft 115 may rotate stably without shaking during the rotation scan, and the through-type shaft 115 is coupled to the inner ring of the ball bearing module 114 and rotates smoothly therewith. That is, the base frame 120 may be connected to the through-type shaft 115 through the ball bearing module 114 so that the through-type shaft 115 is rotatable. The first coil part 113-1 of the rotary transformer 113, in which two sets of coil parts are paired, is physically connected to the through-type shaft 115 and may rotate together with the through-type shaft 115, and the second coil part 113-2 is connected to the base frame 120 and does not rotate. In addition, the first coil part 113-1 that may rotate together with the through-type shaft 115, the shaft electric wire 118 inside the shaft electrically connected to the first coil part 113-1, and the driving unit-side optical fiber 111 that is provided as a separate element inside the through-type shaft 115 may also rotate together during rotation. For reference, the shaft electric wire 118 inside the shaft is illustrated as two wires for convenience in the drawing, and the shaft electric wire 118 inside the shaft may have, for example, a coaxial cable shape. One of the two wires may be electrically connected to one end of the coil of the first coil part 113-1, and the other of the two wires may be electrically connected to the other end of the coil of the first coil part 113-1. When the shaft electric wire 118 inside the shaft has a coaxial cable shape, one of the coaxial core part and the shield part may be electrically connected to one end of the coil of the first coil part 113-1, and the other may be electrically connected to the other end of the coil of the first coil part 113-1.

Likewise, the micro coaxial cable 215 provided in the pullback shaft 213 and the torque coil 214 connected to the FC/PC connector 212, the probe-side optical fiber 211, and an epoxy filling part 216 filling the interior of the pullback shaft 213 all rotate together.

According to the present disclosure, when the FC/PC connector 212 and the FC/PC adapter 117 are used for the connection of the two elements, a key and a key groove formed in each element may also play the role in transmitting mechanical power without the issue of physical slipping between the two elements during their rotation.

According to the present disclosure, when performing photoacoustic-ultrasonic imaging, in addition to the aforementioned rotation scan, a pullback motion may be added to the rotation scan to achieve three-dimensional image acquisition. A motorized linear stage 140 is an element for this, and according to the present disclosure, the base frame 120 is connected to the upper end of the motorized linear stage 140, so that a motion in which the scanning tip 220 within the probe 200 rotates at a constant speed and simultaneously retracts (i.e., moves to the left along the arrow indicating a linear motion in the drawing) at a constant speed may be added. In this case, a pullback force is transmitted to the FC/PC connector 212 through the through-type shaft 115 and the FC/PC adapter 117 fastened to the FC/PC connector 212, to pullback shaft 213, to torque coil 214, and to scanning tip 220, so that the scanning tip 220 may create a helical motion within the catheter tubing 250 to obtain a three-dimensional image. That is, the through-type shaft 115 may relatively move with respect to the driving unit case 101 in a direction in which the through-type shaft 115 extends or in a direction opposite thereto, by the linear stage 140, together with the driving unit-side optical fiber 111, the driving gear 112, and the base frame 120. Similarly, the pullback shaft 213, the probe-side optical fiber 211, the torque coil 214, and the scanning tip 220 are mechanically connected to the FC/PC adapter 117, and thus may relatively move with respect to the catheter tubing 250, the fastening nut 230, and the radial shaft seal 240 by the pullback force in a direction in which the catheter tubing 250 extends and in a direction opposite thereto. In addition, the FC/PC connector 212 located to face away from the one end of the catheter tubing 250 with respect to the radial shaft seal 240, also moves together with the pullback shaft 213, the probe-side optical fiber 211, the torque coil 214, and the scanning tip 220. This is because the FC/PC connector 212 and the pullback shaft 213 are mechanically connected to each other.

FIG. 1 only includes the core concepts derived from the present disclosure, but a device such as a pulser/receiver commonly used in the relevant field may be additionally included in the driving unit 100, and an additional controller and driver device for controlling the device may also be appropriately added.

### Mode for the Invention

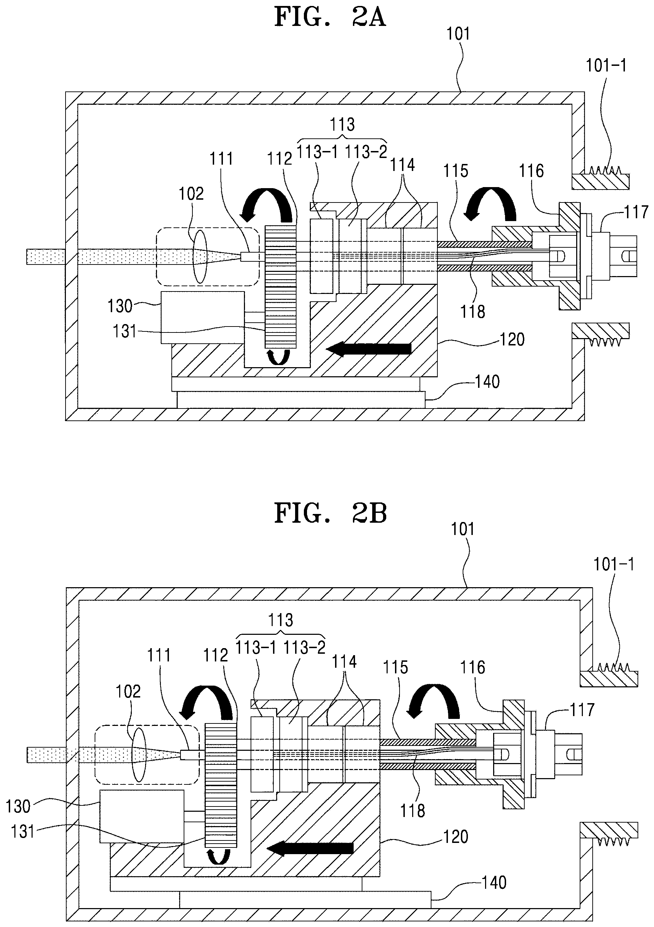

FIGS. 2A and 2B are schematic diagrams showing the internal state of the driving unit included in the helical scan photoacoustic-ultrasonic endoscope of FIG. 1 before and after the pullback motion. FIG. 2A is a schematic diagram showing the internal state of the driving unit 100 before the pullback according to the above-described method, and FIG. 2B is a schematic diagram showing the internal state of the driving unit 100 after the pullback according to the above-described method. The names and roles of the involved elements have been already explained with reference to FIG. 1, and thus, further explanation is omitted. As shown in FIG. 2B, it may be seen that, as the pullback is completed, the base frame 120 attached to the top of the motorized linear stage 140 and elements fastened to the base frame 120 are all moved to the left with respect to the driving unit case 101. Referring to FIG. 2, it is preferable that the optical input unit 102 that may be implemented to be connected to the base frame 120 is implemented so that the optical input unit 102 moves together with the base frame 120 during the pullback motion, but does not rotate together with the driving gear 112 and the driving unit-side optical fiber 111.

In addition, it is possible to obtain a three-dimensional image by attaching the entire probe of the photoacoustic-ultrasonic endoscope to a motorized linear stage that may be provided separately from a corresponding device and then retracting the entire probe at a constant speed. However, in this case, the catheter sweeps over the tissue to be examined, which physically distorts the behavior of the tissue to be examined, or, when scanning a sensitive target such as cancer tissue, causes serious damage to the cancer tissue and spreads cancer cells to other parts. In addition, because the corresponding device is generally used for examination through a narrow and curved tool channel of a video endoscope or electronic endoscope commonly used in the field of gastrointestinal endoscopy, the physical resistance and friction that occur between the outer part of the probe, i.e., the catheter tubing, and the instrument channel of the video endoscope during the relevant procedure makes it difficult to retract the probe at a constant speed, thereby ultimately resulting in the inability to obtain an accurate three-dimensional image.

However, in the case of the photoacoustic-ultrasonic endoscope according to the present disclosure, the position of the catheter tubing 250 is fixed, and the FC/PC connector 212, the pullback shaft 213, the torque coil 214, and the scanning tip 220 perform a pullback motion within the catheter tubing 250, that is, while the catheter tubing 250 is stationary with respect to the tissue to be examined, only the internal elements such as the torque coil 214 and the sensor perform a rotational motion, and a sliding and retracting motion with respect to a radial shaft seal, that is, a helical motion, thereby enabling the obtainment of a desired three-dimensional image. Thus, it is possible to obtain an accurate three-dimensional image while minimizing physical deformation of the tissue to be examined and minimizing damage to the tissue to be examined.

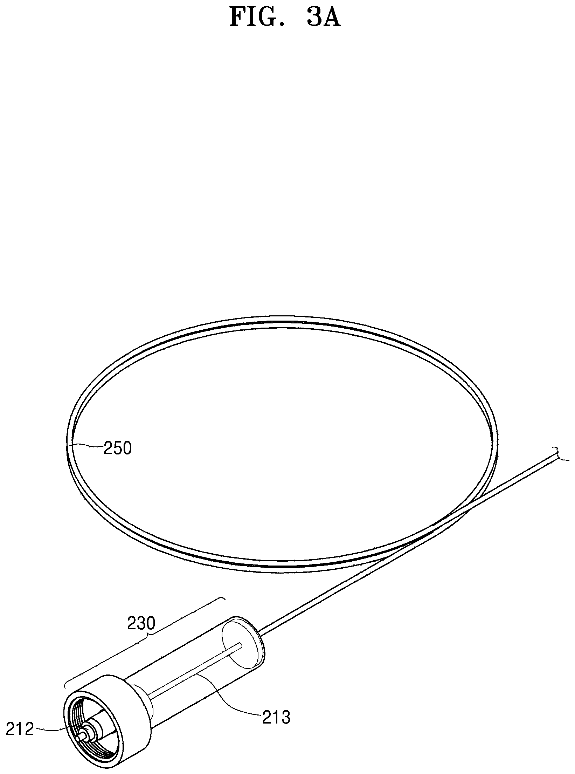

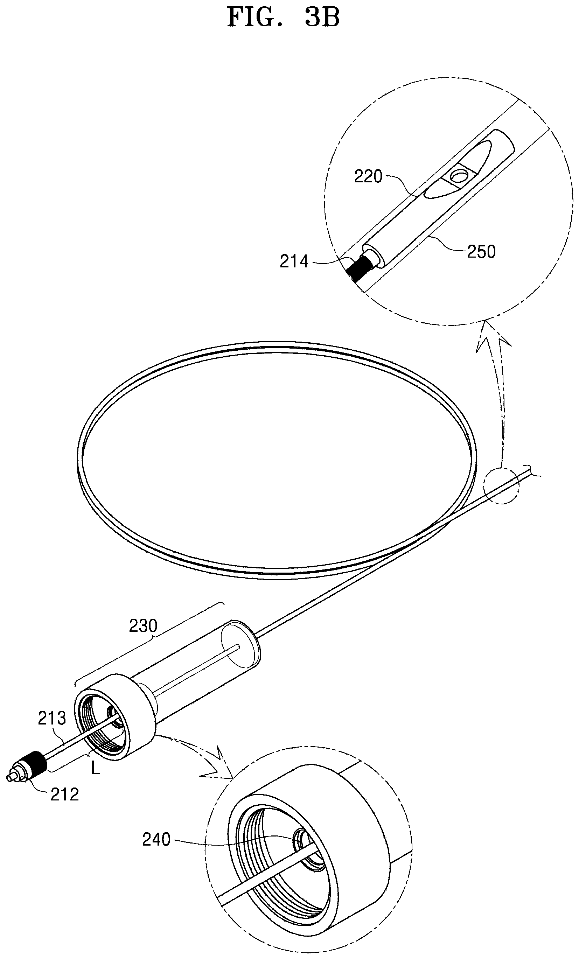

Of course, it would be also possible to consider performing a three-dimensional scan by slightly modifying the catheter tubing of the conventional probe in such a way that a certain range of the end section of the catheter tubing is opened and another tubing, which has one end blocked and has a length with the same extent as the above opened section, is additional overlaid onto the catheter tubing. However, in this case, the fluid filled in the catheter for acoustic matching may not be stably contained, and the matching fluid may eventually leak out during the actual procedure, thereby occurring a very disadvantageous problem, such as hindering stable signal acquisition and prolonging the relevant procedure. However, in the case of the photoacoustic-ultrasonic endoscope according to the present disclosure, such a problem may be effectively prevented by using the radial shaft seal 240 as described below with reference to FIGS. 3A and 3B.

Hereinafter, a detailed description will be given with reference to FIGS. 3A and 3B, which are perspective views showing the probe 200 included in the helical scan photoacoustic-ultrasonic endoscope of FIG. 1 before and after pullback, respectively. A detailed description of the name and role of each element is omitted, and it may be understood that reference numerals indicated in the two drawings correspond to the elements with the same reference numerals presented in FIG. 1.

When the probe 200 is fastened to the driving unit 100 and used for an actual related procedure, each element initially in the position presented in FIG. 3A moves to the position shown in FIG. 3B after rotation and pullback scanning. The two enlarged drawings presented together in FIG. 3B show the shapes of a fastening nut 230 and a scanning tip 220, respectively, and a radial shaft seal 240 provided on the central axis of the fastening nut 230 prevents leakage of a matching fluid 260, which fills the interior of a catheter tubing 250, during the rotation and pullback motion of the pullback shaft 213, torque coil 214, and scanning tip 220. That is, the radial shaft seal 240 may shield the interior of the catheter tubing 250 from the outside.

As shown in the examples of FIGS. 2 and 3, it may be seen that the stroke of the motorized linear stage 140 and the length of the pullback shaft 213 determine the pullback distance of the relevant probe, and the pullback length may be appropriately set and implemented according to a desired application.

Meanwhile, endoscopic procedures of a type similar to that pursued by the present disclosure should generally be completed within 10 minutes, which suggests that the high-efficiency & high-precision optical coupling process required for photoacoustic imaging should be achieved within a much shorter time, and when considering these comprehensive requirements, it is easy to guess how challenging it is to implement a helical scan photoacoustic-ultrasonic endoscope that allows interchangeability between the driving unit and the probe.

According to the present disclosure, when a helical scan photoacoustic-ultrasonic endoscope device is implemented based on the FC/PC connector 212 and the pullback shaft 213 connected thereto, when the probe 200 is fastened to the driving unit 100, the FC/PC connector 212 may be easily pushed into or pulled out of the fastening nut 230, thereby providing convenience and speed during the mounting process, and as a result, the time required for the procedure may be secured much more.

Above all, when a photoacoustic-ultrasonic endoscope capable of helical scanning and connection/disconnection between the probe and the driving unit is implemented according to the present disclosure, the connection part of the probe may be implemented much lighter and more compact than that of the prior art document 4, so that even when the probe is dropped during the procedure, the probability of damage may be greatly reduced, and a probe replacement time may be shortened and thus the related procedure may be performed more smoothly. In addition, the unit cost of probe production may be expected to be significantly reduced.

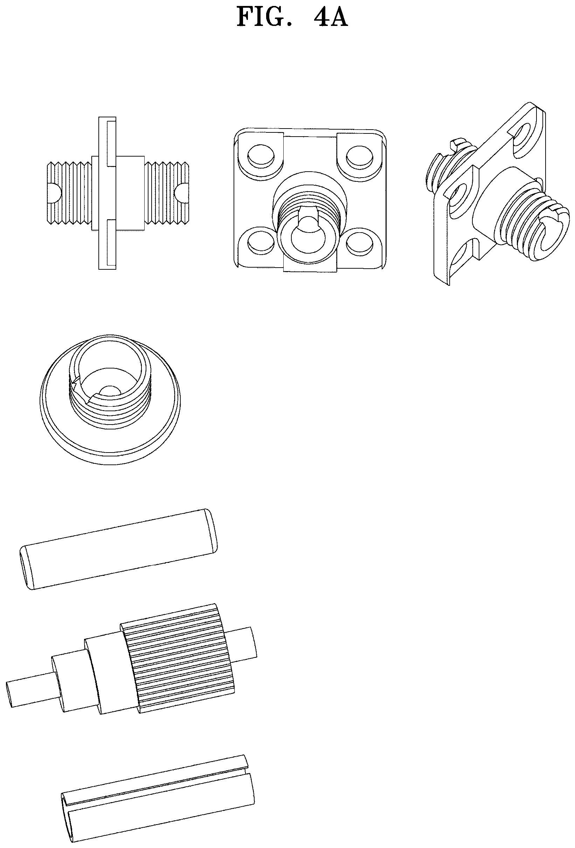

FIG. 4A is a perspective view schematically illustrating optical fiber-related components that may be used in the optical communication field, and shows how to achieve a fast electrical and optical connection path between the probe 200 and the driving unit 100.

It is contemplated to utilize various types of FC/PC adapters, such as those illustrated in the first and second rows of FIG. 4A, and ceramic ferrules and related connectors that may be easily applied to properly fix and terminate an optical fiber at the end of the optical fiber. The FC/PC connector illustrated in the fourth row of FIG. 4A is an example of a related element that may be fastened to an FC/PC adapter, such as those illustrated in the first and second rows of FIG. 4A. The FC/PC connector includes a precisely machined ceramic ferrule, such as that illustrated in the third row of FIG. 4A, at an end of the FC/PC connector, and thus, an optical fiber mounted thereon is precisely positioned in the center of the ferrule. That is, a ceramic ferrule 212-1 in FIG. 4C has a center hole through which the probe-side optical fiber 211 may pass.

The dedicated ferrule for optical fiber provides the tremendous convenience that, when two different optical fiber cables terminated with FC/PC connectors are connected to each other using the "FC/PC to FC/PC adapter" illustrated in the first row of FIG. 4A, the central axes of the two optical fibers are aligned with each other within a considerable level of precision as soon as the two cables are fastened to each other. This is because, as soon as a part of the body of the precisely machined ferrule is inserted into the FC/PC adapter by a split mating sleeve which is included in the FC/PC adapter and is shown in the last row of FIG. 4A, the ferrule and the FC/PC adapter are fitted to each other very tightly. The split mating sleeve, which plays a key role in the alignment process, is made of bronze or ceramic and has a shape on one surface as if the one surface is cut with a slitting saw. When two different ferrules enter into the split mating sleeve to face each other, the sleeve forms close physical contact with the two ferrules due to its elasticity and also ensures that the central axes of the two ferrules may be well aligned with each other.

FIGS. 4B to 4G are schematic diagrams showing the principles of optical coupling and electrical path formation between the probe and the driving unit included in the helical scan photoacoustic-ultrasonic endoscope of FIG. 1.

A photoacoustic-ultrasonic endoscope according to an embodiment of the present disclosure may include a FC/PC to FC/PC adapter (the adapter illustrated in the first row of FIG. 4A) including the split mating sleeve mentioned above, a ceramic ferrule, and an FC/PC connector including the ceramic ferrule. FIGS. 4B and 4C are schematic diagrams each depicting a method of forming an optical path coupling and an electrical path between the probe 200 and the driving unit 100 presented in FIG. 1, based on the elements mentioned above, according to an embodiment.

First, in terms of the connection principle of the optical path, as illustrated in FIG. 4B, the driving unit-side optical fiber 111 mentioned with reference to FIG. 1 is inserted into the through hole of the FC/PC adapter 117 and passes through the center hole of a driving unit-side ceramic ferrule 111-1, and accordingly may be very precisely aligned, within the FC/PC adapter 117, with the probe-side optical fiber 211 inserted into the ceramic ferrule 212-1 in the FC/PC connector 212 included in the probe 200. That is, simply by plugging the FC/PC connector 212 into the FC/PC adapter 117, a part of the driving unit-side ferrule 111-1 and a part of the ceramic ferrule 212-1 of the probe 200 are inserted into a split mating sleeve 117-2 (see FIG. 4C), so that the probe-side optical fiber 211 is very precisely optically aligned with the driving unit-side optical fiber 111 in the center hole of the driving unit-side ceramic ferrule 111-1. In fact, this method is commonly applied to connecting two different single mode optical fibers, and a high level of optical coupling may be achieved even with a very simple method illustrated in FIG. 4B. A part indicated by the reference numeral "212-3" in the drawing refers to a nut part of the FC/PC connector 212, and only the nut part is visible when the FC/PC connector 212 is fastened to the FC/PC adapter 117.

An electrical path while using an FC/PC connector and adapter may be implemented, as shown in FIG. 4C, by manufacturing a conventional FC/PC connector and adapter and a ceramic ferrule with slight modification or processing from the conventional shape. FIG. 4C may be understood as a cross-sectional view taken along the central axis of FIG. 4B, and the FC/PC adapter 117 may include a split mating sleeve 117-2 into which a part of the driving unit-side ferrule 111-1 is inserted and having electrical conductivity, an adapter frame 117-1 having electrical conductivity and surrounding at least a part of the driving unit-side ferrule 111-1, and an adapter insulating member 117-3 interposed between the split mating sleeve 117-2 and the adapter frame 117-1 to electrically insulate the split mating sleeve 117-2 from the adapter frame 117-1. In addition, the FC/PC adapter 117 may further include an electric wire 117-4 inside the FC/PC adapter, etc. One of the core part and the shield part of the shaft electric wire 118 inside the shaft described above may be electrically connected to the adapter frame 117-1, and the other may be electrically connected to the split mating sleeve 117-2 via the electric wire 117-4 inside the FC/PC adapter.

The roles of each element will be explained with reference to FIG. 4C. The coaxial cable 215 includes a coaxial core part 215-1 and a shield part 215-2 and is illustrated as two wires in FIG. 4B and FIG. 4C for convenience. The surface of the ceramic ferrule 212-1 inside the FC/PC connector 212 which is included inside the probe 200 and is electrically connected to the core part 215-1 of the micro coaxial cable 215 is subjected to a conductive treatment by a method such as plating, deposition, or sputtering. Thus, when the ferrule is inserted into the split mating sleeve 117-2 that is also subjected to a conductive treatment and is located inside the FC/PC adapter 117, an electrical connection is automatically formed between them, and furthermore, such electrical connection is continued to a core wire of the shaft electric wire 118 within the through-type shaft 115 through the electric wire 117-4 within the FC/PC adapter, which is connected to the surface of the split mating sleeve 117-2 by a soldering method.

The shield part 215-2 of the micro coaxial cable 215 is connected to an FC/PC connector housing 212-2 that is a part of the FC/PC connector 212, has electrical conductivity, and surrounds at least a part of the ceramic ferrule 212-1, and thus is connected to the FC/PC adapter frame 117-1 that is also physically fastened to the FC/PC connector housing 212-2 and surrounds at least a part of the ceramic ferrule 212-1, through the FC/PC connector nut 212-3 that has electrical conductivity while physically contacting the FC/PC connector housing 212-2, and the FC/PC adapter frame 117-1 is connected to the shield part of the shaft electric wire 118 within the shaft, and thus is electrically connected to an external device.

That is, the FC/PC connector according to the present disclosure is different from the conventional one in that the ceramic ferrule included therein has electrical conductive characteristics, and through this, precise optical coupling (optical alignment) as well as electrical coupling may be realized simultaneously, so that when used, the two paths may be very quickly connected to each other simply by fastening the fastening nut 212-3 of the FC/PC connector 212 to the FC/PC adapter frame 117-1, and as a result, more time may be secured for the related procedure. In addition, because the FC/PC connector 212 has a fastening nut 212-3 and a key groove, when fastened to the FC/PC adapter frame 117-1, there is no delay or loss of rotational power and a fastened part does not come off during a pullback process. In addition, for electrical insulation between the ferrule 212-1 and the housing 212-2 of the FC/PC connector, it is preferable that an insulating member 212-4 is interposed between the ferrule 212-1 and the housing 212-2. In the case of the FC/PC adapter 117, because electrical insulation is also required inside the FC/PC adapter 117, it is preferable that the FC/PC adapter 117 includes an insulating member 117-3 interposed between the split mating sleeve 117-2 and the adapter frame 117-1 to electrically insulate the split mating sleeve 117-2 from the adapter frame 117-1.

In addition to the method of using a ceramic ferrule 212-1 subjected to the conductive treatment exemplified in FIG. 4C, an electrical connection method between the FC/PC adapter 117 and the FC/PC connector 212 may be implemented by including an additional conductive element having a tube or ring shape, which is electrically insulated from the FC/PC connector housing 212-2, on a part of the surface of the ceramic ferrule 212-1 or around the ceramic ferrule 212-1, and in this case, an element such as a spring that provides elasticity around the ceramic ferrule 212-1 for closer contact of the connection point may also be added. The conductive element having a tube or ring shape is an element that has to be electrically connected to the core part 215-1 of the micro coaxial cable 215.

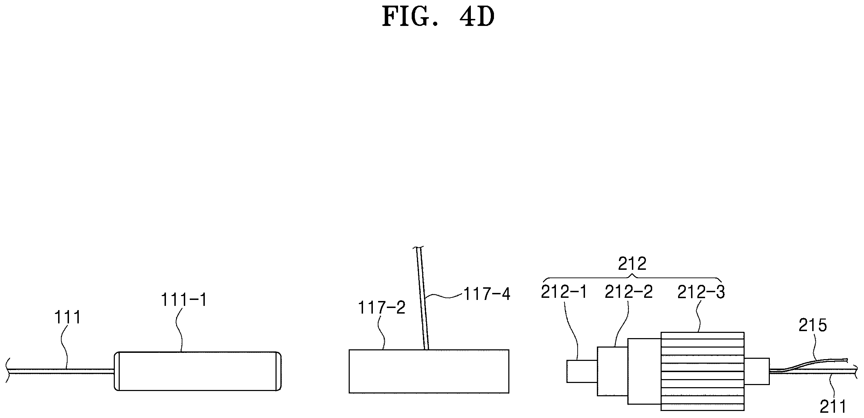

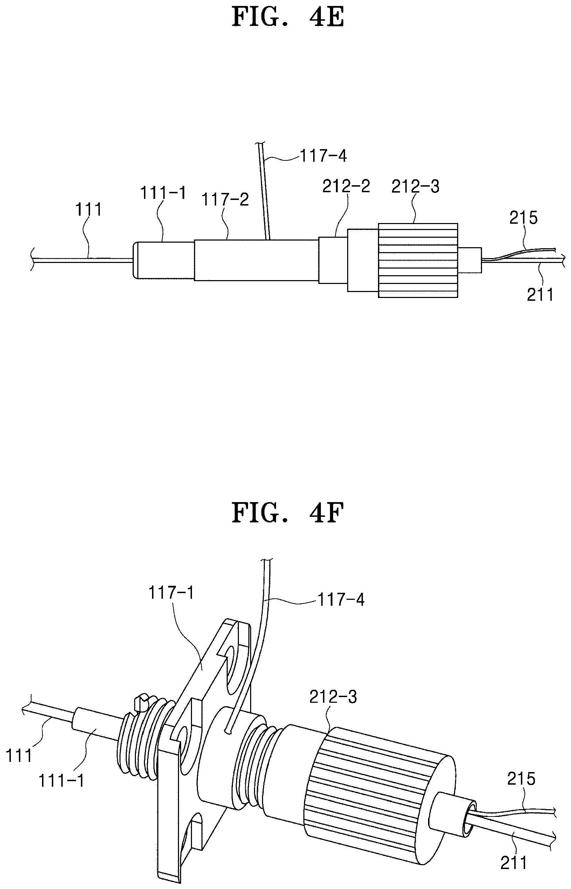

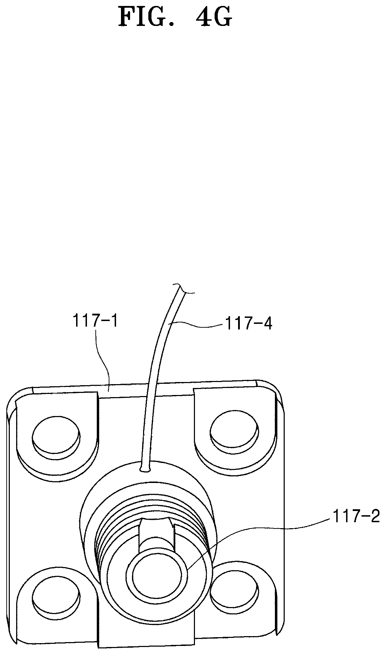

FIG. 4D shows the external appearance of each unit of the core elements illustrated in FIG. 4C based on examples that have actually been implemented, FIG. 4E is a view showing the relationship in which the core elements actually interlock with each other when fastened to each other, and FIG. 4F is a view when the FC/PC adapter 117 is included, and the view may be understood as the external appearance when FIG. 4C is actually implemented. In this case, the FC/PC adapter 117 is different from the typical FC/PC adapter (see the example of FIG. 4A) in that the FC/PC adapter 117 includes the conductive split mating sleeve 117-2 as described above (see FIG. 4G).

A method of forming an electrical path as well as optical coupling when fastening the probe 200 and the driving unit 100 to each other based on an FC/PC connector, an adapter, and a ceramic ferrule has been described above. The basic forms of the FC/PC connector, the adapter, and the ceramic ferrule that may be applied to the present disclosure may be much more diverse than those exemplified in the present specification. In any case, the present disclosure discloses a method of simply realizing two connection paths based on these conventional elements, and thus the key feature is that the sizes of the relevant parts may be significantly reduced.

The ceramic ferrule applicable to the present disclosure may include Thorlabs' CF270-10 having a diameter of Φ2.5 mm, and does not necessarily have to be made of ceramic material. In addition, the diameter of the ceramic ferrule is not limited to 2.5 mm and may be changed as needed.

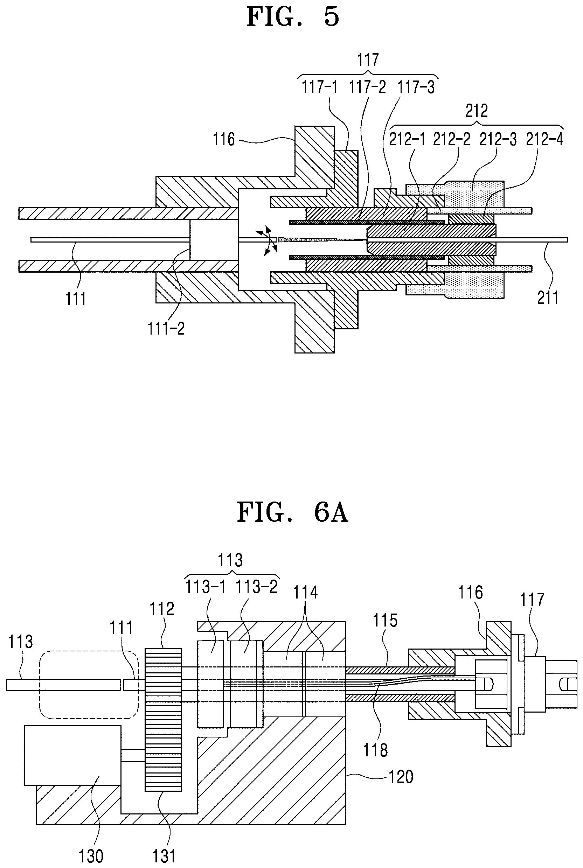

A piezoelectric positioner 111-2 may be additionally provided in the through-type shaft 115 of a helical scan photoacoustic-ultrasonic endoscope according to another embodiment. In FIG. 4, a method of achieving optical coupling based on a conventional FC/PC connector and adapter is illustrated, but when the core thickness of the optical fiber used in the probe is very small, it may be difficult to achieve a very high level of optical coupling efficiency at a fastening point with only the presented method. In this case, as shown in FIG. 5, which is a schematic diagram showing a method of achieving high-efficiency optical coupling in a helical scan photoacoustic-ultrasonic endoscope according to an embodiment of the present disclosure, by adding the piezoelectric positioner 111-2 within the through-type shaft 115 and performing an automatic optical alignment process between the driving unit-side optical fiber 111 and the probe-side optical fiber 211 before using the photoacoustic-ultrasonic endoscope based on the piezoelectric positioner 111-2. Through this alignment process, the optical coupling efficiency between the driving unit-side optical fiber 111 and the probe-side optical fiber 211 may be further increased.

In general, a ceramic ferrule is made of a light-scattering material, and thus, when light does not enter a center hole of the ceramic ferrule and enters the periphery of the ceramic ferrule, light is scattered due to light scattering. Therefore, when the intensity of this scattered light (i.e., light that does not proceed to the probe-side optical fiber 211 as originally intended but is backscattered) is measured by a separate optical sensor (not shown) placed around the piezoelectric positioner 111-2 while adjusting the direction of light emission of the driving unit-side optical fiber 111 inserted into the central axis of the piezoelectric positioner 111-2, a higher optical coupling may be achieved. In addition, an additional optical focusing member (not shown), such as a GRIN or ball lens, may be attached to the end of the driving unit-side optical fiber 111 to more efficiently focus the laser beam emitted from the driving unit-side optical fiber 111 on the entrance of the probe-side optical fiber 211.

In this way, the automatic optical alignment process using the piezoelectric positioner 111-2 may be implemented in a form in which elements (not shown) required for the relevant procedure are automatically connected to and automatically separated from the piezoelectric positioner 111-2 through a predetermined electrical path before using the device. Electrical signals and power that may be required in this process may also be transmitted using a rotary transformer.

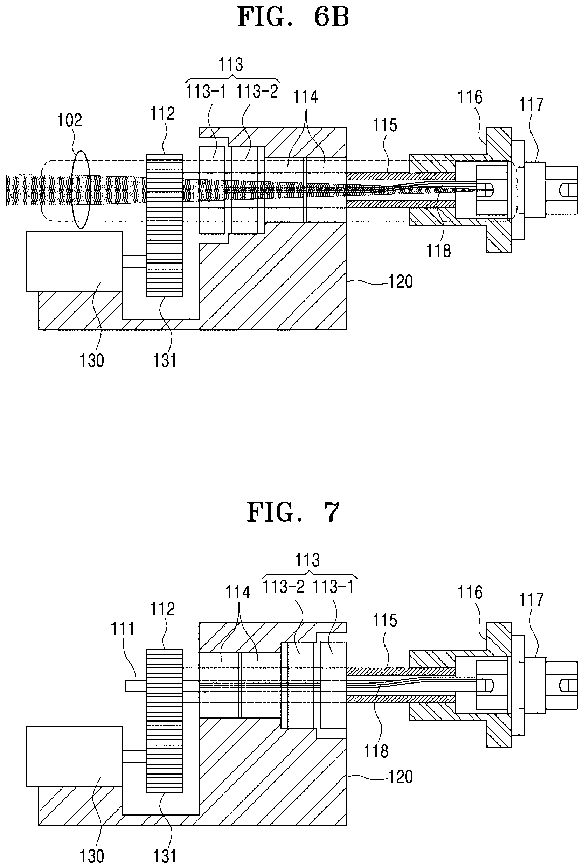

FIGS. 6A and 6B are schematic diagrams showing a method of implementing an optical input unit in a helical scan photoacoustic-ultrasonic endoscope according to an embodiment of the present disclosure. An optical rotary joint (indicated by the dotted line near the optical input unit 102 in FIGS 1, 2A, and 2B) of a helical scan photoacoustic-ultrasonic endoscope according to another embodiment may be implemented in various forms, as shown in the example presented by the dotted line in FIGS. 6A and 6B.

Referring to FIGS. 6A, a fiber-type optical input unit 103, which transmits light to the driving unit-side optical fiber 111 while being positioned very close to the driving unit-side optical fiber 111 that may rotate, but does not rotate unlike the driving unit-side optical fiber 111, may be used to implement the optical rotary joint. In this case, a GRIN or ball lens, etc. may be added to the end of the optical input unit 103, as mentioned above.

Furthermore, as shown in FIG. 6A, the interior of the through-type shaft 115 may be made to have a hollow shape in which light may freely travel, so that light may be propagate into the through-type shaft 115 from a distance. Because the length of the base frame 120 that may be implemented according to the present disclosure may be considerably short, such configuration may be implemented without difficulty while maintaining a desired optical numerical aperture. When this method is implemented, the beam diameter at the focal point may be formed to be larger than the core diameter of the probe-side optical fiber 211, and thus, the desired optical coupling (or optical transmission) may be achieved even when the probe-side optical fiber 211 is not exactly placed on the central axis of rotation. In addition, when the optical rotary joint is implemented in the form of FIG. 6B, the accuracy of an optical input may be improved by adding the piezoelectric positioner mentioned in FIG. 5 before the optical input unit 102. In this case, because the optical input unit 102 does not rotate at all during the process of using the endoscope device, there is an additional advantage in that the light emission direction may be freely controlled even during image acquisition (i.e., during the rotational and translational motion of the probe).

FIG. 7 is a schematic diagram showing the possibility of changing the position of a rotary transformer in a helical scan photoacoustic-ultrasonic endoscope according to an embodiment of the present disclosure. As shown in FIG. 7, the position of a rotary transformer 113 of a helical scan photoacoustic-ultrasonic endoscope according to an embodiment may be changed depending on the application.

Referring to FIG. 7, the position of the rotary transformer 113 of the helical scan photoacoustic-ultrasonic endoscope according to an embodiment may be implemented to be moved to the right side of a ball bearing module 114, in which case only a part 113-1 of the drawing rotates. Furthermore, as in the prior art document 4, the position of the rotary transformer 113 may be moved to the outside of a driving gear 112, that is, to the left side of the driving gear 112 in FIG. 7.

Although the present disclosure has been described with reference to the embodiments shown in the drawings, the embodiments are merely exemplary, and those skilled in the art will understand that various modifications and equivalent other embodiments may be made from the embodiments. Therefore, the true technical protection scope of the present disclosure should be determined by the technical idea of the appended claims.

### Industrial Applicability

Within a very light and compact size, even when applying a single-mode optical fiber, it is possible to quickly achieve very stable and precise optical coupling at an optical coupling point, and thus, related procedures may be performed more conveniently and smoothly, and three-dimensional images obtained therethrough may be much more accurate and reliable.

## Claims

1. A helical scan photoacoustic-ultrasonic endoscope comprising:
a probe; and
a driving unit providing rotational power and pullback translational motion to the
probe,
wherein the probe comprises:
a catheter tubing extending in one direction and having an interior that is hollow,
one end of the catheter tubing being closed and the other end of the catheter tubing being open;
a fastening nut that is fastened to the other end of the catheter tubing and has
a through hole corresponding to the interior of the catheter tubing;
a pullback shaft extending into the interior of the catheter tubing through the through hole of the fastening nut and having an interior that is hollow; and
a radial shaft seal interposed between the fastening nut and the pullback shaft and surrounding the pullback shaft around a central axis of the through hole of the fastening nut.

2. The helical scan photoacoustic-ultrasonic endoscope of claim 1, wherein the driving unit comprises a linear stage and a rotary transformer, mechanically connected to the pullback shaft.

3. The helical scan photoacoustic-ultrasonic endoscope of claim 1, wherein the radial shaft seal shields the interior of the catheter tubing from the outside.

4. The helical scan photoacoustic-ultrasonic endoscope of claim 1, further comprising:
a probe-side optical fiber passing through the interior of the pullback shaft and extending in a direction toward the one end of the catheter tubing;
a torque coil surrounding the probe-side optical fiber, and passing through the interior of the pullback shaft and extending in the direction toward the one end of the catheter tubing; and
a scanning tip connected to a part of the torque coil in the direction toward the one end of the catheter tubing.

5. The helical scan photoacoustic-ultrasonic endoscope of claim 4, wherein the pullback shaft, the probe-side optical fiber, the torque coil, and the scanning tip are relatively movable with respect to the catheter tubing, the fastening nut, and the shaft seal, in a direction in which the catheter tubing extends and in a direction opposite thereto.

6. The helical scan photoacoustic-ultrasonic endoscope of claim 4, further comprising a matching fluid filling the interior of the catheter tubing.

7. The helical scan photoacoustic-ultrasonic endoscope of claim 4, further comprising a connector connected to an end of the pullback shaft in a direction facing away from the one end of the catheter tubing.

8. The helical scan photoacoustic-ultrasonic endoscope of claim 7, wherein the connector is located in the direction facing away from the one end of the catheter tubing, with respect to the shaft seal.

9. The helical scan photoacoustic-ultrasonic endoscope of claim 7, further comprising a micro coaxial cable electrically connected to the scanning tip and extending to the connector through the interior of the torque coil,
wherein the connector comprises:
a ferrule having a central hole through which the probe-side optical fiber passes;
a connector housing surrounding at least a part of the ferrule and having electrical conductivity;
a connector nut physically contacting the connector housing and having electrical conductivity; and
a connector insulating member interposed between the ferrule and the connector housing to electrically insulate the ferrule from the connector housing,
wherein a core part of the micro coaxial cable is electrically connected to the ferrule, and a shield part of the micro coaxial cable is electrically connected to the connector housing.

10. The helical scan photoacoustic-ultrasonic endoscope of claim 9, wherein the driving unit comprises an adapter,
wherein the adapter comprises:
a split mating sleeve into which a part of the ferrule is inserted;
an adapter frame surrounding at least a part of the ferrule and having electrical conductivity; and
an adapter insulating member interposed between the split mating sleeve and the adapter frame to electrically insulate the split mating sleeve from the adapter frame.
